# EUROPEAN PATENT APPLICATION

(11) **EP 2 113 556 A2**
(43) Date of publication of application: **04.11.2009**
(21) Application number: 09166152.0
(22) Date of filing: 20.12.2006
(51) Int. Cl.: C11D 1/00, C11D 1/02, C11D 1/38, C11D 1/65, C11D 3/00, C11D 3/20

(54) **Foamable alcoholic composition**

(30) Priority: 28.12.2005 US 754536; 22.05.2006 US 438664
(62) Divisional of application: 06256249.1
(71) Applicant: Gojo Industries, Inc., Akron, OH 44311 (US)
(72) Inventor: Snyder, Marcia, Stow, OH 44224 (US); Viscovitz, John H., Akron, OH 44319 (US)
(74) Representative: Makovski, Priscilla Mary

(57) **Abstract**

A stable foam composition includes greater than about 40 weight percent of an alcohol, based upon the total weight of the alcoholic composition, and a foaming surfactant selected from fluorosurfactants, siloxane polymer surfactants, and combinations thereof. When the foaming surfactant includes a fluorosurfactant, the alcoholic composition further includes a polymeric or oligomeric foam stabilizer.

## Description

This application gains priority from U.S. Provisional Application No. 60/754,536, filed December 28, 2005, which is incorporated herein by reference.

### TECHNICAL FIELD

This invention relates to foamable alcoholic compositions, and more particularly, to foamable alcoholic compositions that include a foaming surfactant selected from fluorosurfactants, siloxane polymer surfactants, and combinations thereof.

### BACKGROUND OF THE INVENTION

Foam cleaning products are popular, in part because they are easier to spread on surfaces. Consumers seem to prefer the luxury of foamed soap products. Less foam is needed to produce the same cleaning power as liquids or gels, due at least partly to the higher surface area of the foam. Properly formulated foam products do not produce the drip and splash that is experienced with traditional gelled or liquid products, due to the higher surface tension of the foam. This prevents damage to the floors and walls of facilities where the product dispensers are used. Manufacturing of foam products may be easier than gelled products, which often incorporate powdered thickeners that are difficult to handle.

Aqueous foam cleaning products have been described, for example, a foam cleaning composition containing a surfactant, a foam-boosting solvent such as a glycol, and at least 80 percent by weight (wt. %) water.

Alcoholic products are popular as sanitizers for the skin. However, foaming products based upon alcoholic compositions are problematic, because alcohol is known to have strong defoaming properties. Although aerosol-based alcoholic foams are available, these aerosol products are generally more expensive to manufacture than non-aerosol foams.

A stable foamed composition containing water and an alkoxylated silicone compound has been described that may contain up to 30 wt. % alcohol, and may be stable for one month or longer. However, alcoholic compositions do not exhibit effective antimicrobial properties unless the alcohol is present in an amount of 50 weight percent or more.

A foamable liquid cleanser containing a surfactant, a humectant, water, and from about 35 to about 70 wt. % alcohol has been described, however the foam produced when this composition is aerated collapses substantially to liquid within about 5 to 45 seconds.

A skin disinfecting formulation has been described that comprises: (a) an alcohol in an amount from about 50 to about 80 weight percent of the total composition; (b) from about 0.02 wt. % to about 5 wt. % of a block copolymer; (c) from about 5 wt. % to about 25 wt. % of a foaming surfactant; (d) from about 0 wt. % to about 3 wt. % of a thickening agent; (e) from about 1 wt. % to about 5 wt. % of an emulsifier; (f) from about 0wt. % to about 5wt. % of a preservative; (g) from about 1 wt. % to about 10 wt. % of a cleaning agent; (h) from about 0.5 wt. % to about 5 wt. % of a polyalkylene glycol; (i) from about 0.05 wt. % to about 5 wt. % of a moisturizer and/or emollient; and (j) from about 6 wt. % to about 30 wt. % of water. The foaming surfactants that are taught are ammonium fatty sulfo succinate, cocamide dea, alkonolamides such as cocodiethanolamide, amine oxides such as cetyldimethyl amino oxide and amphoterics such as isostearoamphoropionate and lauramidopropyl betaine surfactant.

Commercially available non-aerosol alcoholic foams have been formulated with specific perfluoroalkyl phosphate surfactants and dispensed from a foaming pump mechanism. But the foam thus produced is fleeting, and will immediately begin to deflate or liquefy into a water-thin liquid. When the alcoholic foam is dispensed from a common foaming pump mechanism, some of the foam is left within the tubes of the dispenser, and, as the foam breaks down, the composition often drips out of the dispenser. This dripping not only wastes product, it makes the area around the dispenser look unsanitary and may result in damage to the walls and floors, as well as resulting in a slip hazard if the product is dripping on the floor. Thus, there is a need for stabilized foamable alcoholic compositions.

### SUMMARY OF THE INVENTION

One or more embodiments of this invention provide a foamable alcoholic composition comprising at least about 40 weight percent alcohol, based upon the total weight of the alcoholic composition; and a foaming surfactant selected from the group consisting of siloxane polymer surfactants, anionic, cationic, and zwitterionic fluorosurfactants, and combinations thereof; with the proviso that when the foaming surfactant comprises a fluorosurfactant, the foamable alcoholic composition further comprises a polymeric or oligomeric foam stabilizer.

One or more embodiments of this invention further provide an antimicrobial composition comprising at least about 40 weight percent alcohol, based upon the total weight of the alcoholic composition; and a foaming surfactant selected from the group consisting of siloxane polymer surfactants, anionic, cationic, and zwitterionic fluorosurfactants, and combinations thereof; with the proviso that when the foaming surfactant comprises a fluorosurfactant, the foamable alcoholic composition further comprises a polymeric or oligomeric foam stabilizer.

One or more embodiments of this invention still further provide a method for forming a stabilized alcoholic foam, the method comprising combining an alcohol, a foaming surfactant selected from the group consisting of siloxane polymer surfactants, anionic, cationic, and zwitterionic fluorosurfactants, and combinations thereof, to form a foamable alcoholic composition; mixing said alcoholic composition and air or an inert gas in a mixing chamber to form a mixture; and passing said mixture through a mesh screen to form a foam, wherein said foamable alcoholic composition comprises at least about 40 percent by weight alcohol, based upon the total weight of the alcoholic composition, and with the proviso that, when the foaming surfactant comprises a fluorosurfactant, the foamable alcoholic composition further comprises a polymeric or oligomeric foam stabilizer.

One or more embodiments of this invention further provide a method for reducing dripping of a composition from a foaming pump dispenser adapted to dispense said composition, the method comprising providing a foamable composition comprising at least about 40 weight percent alcohol, based upon the total weight of the alcoholic composition, and a foaming surfactant selected from the group consisting of siloxane polymer surfactants, anionic, cationic, and zwitterionic fluorosurfactants, and combinations thereof, with the proviso that where the foaming surfactant comprises a fluorosurfactant, the foamable alcoholic composition further comprises a polymeric or oligomeric foam stabilizer; providing a foaming pump dispenser having a dispenser head; and placing the foamable composition into the foaming pump dispenser.

### DETAILED DESCRIPTION OF ILLUSTRATIVE EMBODIMENTS

Foamable alcoholic compositions in accordance with this invention include at least one alcohol and at least one foaming surfactant. In one embodiment, the alcohol is a lower alkanol, i.e. an alcohol containing 1 to 4 carbon atoms. Typically, these alcohols have antimicrobial properties. Examples of lower alkanols include, but are not limited to, methanol, ethanol, propanol, isopropanol, butanol, isobutanol, tertiary butanol, and mixtures thereof. In one embodiment, the alcohol comprises ethanol.

Generally, the alcoholic composition comprises an amount of alcohol of at least about 40 weight percent (wt. %), based upon the total weight of the alcoholic composition. In one embodiment, the alcoholic composition comprises at least about 45 weight percent alcohol, in another embodiment, the alcoholic composition comprises at least about 50 weight percent alcohol, and in yet another embodiment, the alcoholic composition comprises at least about 60 weight percent alcohol, based upon the total weight of alcoholic composition. More or less alcohol may be required in certain instances, depending particularly on other ingredients and/or the amounts thereof employed in the composition. In certain embodiments, the alcoholic composition comprises from about 40 weight percent to about 98 weight percent alcohol, in other embodiments, the alcoholic composition comprises from about 45 weight percent to about 95 weight percent of alcohol, in yet other embodiments, the alcoholic composition comprises from about 50 weight percent to about 90 weight percent of alcohol, and in still other embodiments, the alcoholic composition comprises from about 60 weight percent to about 80 weight percent of alcohol, based upon the total weight of the alcoholic composition.

The foaming surfactant contributes foaming properties to the alcoholic composition, and may include anionic, cationic, nonionic, zwitterionic, or amphoteric surfactants and their associated salts. In one embodiment, the foaming surfactant includes a fluorosurfactant, a siloxane polymer surfactant, or a combination thereof. Fluorosurfactants include compounds that contain at least one fluorine atom. Examples of fluorosurfactants include perfluoroalkylethyl phosphates, perfluoroalkylethyl betaines, fluoroaliphatic amine oxides, fluoroaliphatic sodium sulfosuccinates, fluoroaliphatic stearate esters, fluoroaliphatic phosphate esters, fluoroaliphatic quaternaries, fluoroaliphatic polyoxyethylenes, and the like, and mixtures thereof.

In one embodiment, the fluorosurfactant contains a charged species, *i.e.* is anionic, cationic, or zwitterionic. Examples of fluorosurfactants containing a charged species include perfluoroalkylethyl phosphates, perfluoroalkylethyl betaines, fluoroaliphatic amine oxides, fluoroaliphatic sodium sulfosuccinates, fluoroaliphatic phosphate esters, and fluoroaliphatic quaternaries. Specific examples of fluorosurfactants include DEA-C8-18 perfluoroalkylethyl phosphate, TEA-C8-18 perfluoroalkylethyl phosphate, NH₄-C8-18 perfluoroalkylethyl phosphate, and C8-18 perfluoroalkylethyl betaine.

In one embodiment, the fluorosurfactant includes a compound that may be represented by the formula

[F₃CF₂C-(CF₂CF₂)ₓ-CH₂CH₂-O-P₂O₃]⁻ [R¹]⁺

where [R¹]⁺ includes DEA, TEA, NH₄, or betaine, and where x is an integer from about 4 to about 18.

Siloxane polymer surfactants may be generally characterized by containing one or more Si-O-Si linkages in the polymer backbone. The siloxane polymer surfactant may or may not include a fluorine atom. Therefore, some foaming surfactants may be classified as both fluorosurfactants and siloxane polymer surfactants. Siloxane polymer surfactants include organopolysiloxane dimethicone polyols, silicone carbinol fluids, silicone polyethers, alkylmethyl siloxanes, amodimethicones, trisiloxane ethoxylates, dimethiconols, quaternized silicone surfactants, polysilicones, silicone crosspolymers, and silicone waxes.

Examples of siloxane polymer surfactants include dimethicone PEG-7 undecylenate, PEG-10 dimethicone, PEG-8 dimethicone, PEG-12 dimethicone, perfluorononylethyl carboxydecal PEG 10, PEG-20/PPG-23 dimethicone, PEG-11 methyl ether dimethicone, bis-PEG/PPG-20/20 dimethicone, silicone quats, PEG-9 dimethicone, PPG-12 dimethicone, fluoro PEG-8 dimethicone, PEG 23/PPG 6 dimethicone, PEG 20/PPG 23 dimethicone, PEG 17 dimethicone, PEG5/PPG3 methicone, bis PEG20 dimethicone, PEG/PPG20/15 dimethicone copolyol and sulfosuccinate blends, PEG-8 dimethicone\dimmer acid blends, PEG-8 dimethicone\fatty acid blends, PEG-8 dimethicone\cold pressed vegetable oil\polyquaternium blends, random block polymers and mixtures thereof.

In one embodiment, the siloxane polymer surfactant includes a compound that may be represented by the formula

R²- Si(CH₃)₂-[O-Si(CH₃)₂]*ₐ*[O-Si(CH₃)R³]*_{b}*-O-Si(CH₃)₂-R²

where R² and R³ independently include a methyl group or a moiety that may be represented by the formula

-(CH₂)₃-O-(CH₂CH₂O)*_{c}*-[CH₂CH(CH₃)O]*_{d}*-(CH₂CH₂O)*ₑ*H

with the proviso that both R² and R³ are not CH₃, where a is an integer from about 3 to about 21, b is an integer from about 1 to about 7, c is an integer from about 0 to about 40, d is an integer from about 0 to about 40, and e is an integer from about 0 to about 40, with the proviso that a ≥ 3 x b and that c + d + e ≥ 5.

The amount of foaming surfactant is not particularly limited, so long as an effective amount to produce foaming is present. In certain embodiments, the effective amount to produce foaming may vary, depending upon the amount of alcohol and other ingredients that are present. In one or more embodiments, the alcoholic composition includes at least about 0.002 wt. % of foaming surfactant, based upon the total weight of the alcoholic composition. In another embodiment, the alcoholic composition includes at least about 0.01 wt. % of foaming surfactant, based upon the total weight of the alcoholic composition. In yet another embodiment, the alcoholic composition includes at least about 0.05 wt. % of foaming surfactant, based upon the total weight of the alcoholic composition.

In one embodiment, the foaming surfactant is present in an amount of from about 0.002 to about 4 weight percent, based upon the total weight of the alcoholic composition. In another embodiment, the foaming surfactant is present in an amount of from about 0.01 to about 2 weight percent, based upon the total weight of the alcoholic composition. It is envisioned that higher amounts may also be effective to produce foam. All such weights as they pertain to listed ingredients are based on the active level and, therefore, do not include carriers or by-products that may be included in commercially available materials, unless otherwise specified.

In some embodiments, for economic or other reasons it may be desirable to limit the amount of fluorosurfactant used. Advantageously, stable foam can be produced from a composition according to the present invention containing greater than about 60 wt. % alcohol, and from about 0.002 to about 0.5 wt. % fluorosurfactant, based upon the total weight of the composition. In certain embodiments, the foamable composition includes greater than about 65 wt. % alcohol, and from about 0.002 to about 0.4 wt. % fluorosurfactant, based upon the total weight of the composition.

In other embodiments, it may be desirable to use higher amounts of foaming surfactant. For example, in certain embodiments where the foaming alcoholic composition of the present invention includes a cleansing or sanitizing product that is applied to a surface and then rinsed off, higher amounts of foaming surfactant may be employed. In these embodiments, the amount of foaming surfactant is present in amounts up to about 35 wt. %, based upon the total weight of the composition.

In one or more embodiments, the foaming surfactant is added directly to the alcoholic composition. In other embodiments, the foaming surfactant is added to the alcoholic composition as a solution or emulsion. In other words, the foaming surfactant may be premixed with a carrier to form a foaming surfactant solution or emulsion, with the proviso that the carrier does not deleteriously affect the foaming properties of the alcoholic composition. Examples of carriers include water, alcohol, glycols such as propylene or ethylene glycol, ketones, linear and/or cyclic hydrocarbons, triglycerides, carbonates, silicones, alkenes, esters such as acetates, benzoates, fatty esters, glyceryl esters, ethers, amides, polyethylene glycols and PEG/PPG copolymers, inorganic salt solutions such as saline, and mixtures thereof. It will be understood that, when the foaming surfactant is premixed to form a foaming surfactant solution or emulsion, the amount of solution or emulsion that is added to the alcoholic composition may be selected so that the amount of foaming surfactant falls within the ranges set forth hereinabove.

Foaming surfactant solutions and emulsions are commercially available, for example under the trade names Masurf® FS-115 as a 14 wt. % solution of fluoroaliphatic phosphate ester in water, Masurf® FS-130 as a 28 wt. % solution of fluoroaliphatic phosphate ester in water, available from Mason Chemical Company. As is known in the art, siloxane surfactants are also commercially available.

In certain embodiments, the alcoholic composition of the present invention further includes at least one foam stabilizer. In one embodiment, where the foaming surfactant comprises a fluorosurfactant, the foam stabilizer may be selected from polymeric or oligomeric foam stabilizers. In another embodiment, where the foaming surfactant comprises a siloxane surfactant, the foam stabilizer is optional, and may be polymeric or non-polymeric.
In one embodiment, the foam stabilizer comprises a cationic oligomer or polymer.

Advantageously, the alcoholic composition of these embodiments exhibits improved foam stability when compared to an alcoholic composition without the foam stabilizer. By "foam stability" is meant the length of time that it takes for a foam to break down into a liquid.

Polymeric foam stabilizers include polyquaternium polymers. In general, a polyquaternium polymer is one that is designated as such by the CTFA. Polyquaternium polymers may be characterized by containing a quaternary ammonium group. Non-limiting examples of polyquaterniums include those listed in Table 1, below, including the INCI name and technical name.

**Table 1**

| **INCI Name Polyquaternium-** | **Technical Name** |
|---|---|
| **X** | |
| -2 | Bis(2-chloroethyl)ether, polym. w. N,N'-bis[3-(dimethylamino)propyl]urea |
| -4 | Hydroxyethylcellulose Dimethyldiallylammoinum Chloride Copolymer |
| -5 | Copolymer of acrylamide and beta-methacrylyloxyethyl trimethyl ammonium methosulfate |
| -6 | Polydimethyldiallyl Ammonium Chloride |
| -7 | Dimethyldiallyl Ammonium Chloride & Acrylamide Copolymer |
| -9 | Polydimethyaminoethyl methacrylate quaternized with Methyl Bromide |
| -10 | Hydroxyethylcellulose reacted with trimethyl ammonium substituted epoxide |
| -11 | PVP N,N-Dimethyl Aminoethyl Methacrylic Acid Copolymer Diethyl Sulfate Soln |
| -14 | Ethanaminium, N,N,N-Trimethyl-2-[(2-methyl-1-oxo-2-propenyl)oxy]-, Methyl Sulfate Homopolymer |
| -15 | Acrylamide-Dimethylaminoethyl Methacrylate Methyl Chloride Copolymer |
| -16 | 3-Methyl-1-Vinylimidazolium Chloride-1-Vinyl-2-Pyrrolidinone Chloride |
| -17 | Quat salt made from Adipic acid & diethylaminopropylamine & dichloroether |
| -18 | Quat salt prepared by the reaction of adipic acid and dimethylaminopropylamine, reacted with dichloroethyl ether |
| -19 | Quat ammonium salt prepared by the reaction of polyvinyl alcohol with 2,3- epoxypropylamine |
| -20 | Quat ammonium salt prepared by the reaction of polyvinyl octadecyl ether with 2,3-epoxypropylamine |
| -22 | Acrylic Acid-Diallyldimethylammonium Chloride (DADMAC) Polymer |
| -22 | Acrylic Acid-Diallyldimethylammonium Chloride (DADMAC) Polymer |
| -24 | Polyquat ammonium salt of hydroxyethyl cellulose reacted with lauryl dimethyl ammonium substituted epoxide |
| -27 | Block Copolymer of Polyquaternium-2 and 17 |
| -28 | Vinylpyrrolidone/Methacrylamidopropyltrimethylammonium Chloride Copolymer |
| -29 | Propoxylated Chitosan quaternized with epichlorhydrin |
| -30 | Ethanaminium, N-Carboxymethyl)-N,N-Dimethyl-2-((2-Methyl-1-Oxo-2-Propenyl)Oxy)-, Inner Salt, Polymer with Methyl 2-Methyl-2-Propenoate |
| -31 | 2-propane nitrile reaction product w/ N,N-dimethylpropanediamine, Sulfate |
| -32 | Acrylamide-Dimethylaminoethyl Methacrylate Methyl Chloride (DMAEMA) Copolymer |
| -37 | Trimethylaminoethyl Methacrylate Chloride Polymer |
| -39 | Acrylic Acid (AA), Polymer w/ Acrylamide & Diallyldimethylammonium Chloride(DADMAC) |
| -42 | Polyoxyethylene (dimethyliminio)ethylene-(dimethyliminio)ethylene dichloride |
| -43 | Copolymer of Acrylamide, acrylamidopropyltrimonium chloride, amidopropylacrylamide & DMAPA Monomers |
| -44 | Polyquat ammonium salt of vinylpyrrilidone & quaternized imidazoline monomers |
| -46 | Quat ammonium salt of vinylcaprolactum, vinylpyrrolidone &methylvinylimidazolium |
| -47 | Quat ammonium chloride- acrylic acid, methyl acrylate & methacrylamidopropyltrimonium Chloride |
| -48 | Copolymer of methacryolyl ethyl betaine, 2-hydroxyethylmethacrylate & methacryloylethyltrimethylammonium chloride |
| -51 | 3,5,8-Triox-4-Phosphaundec-10-en-1-aminium, 4-Hydroxy-N,N,N,10-Tetramethyl-9-Oxo, Inner Salt, 4-Oxide, Polymer with Butyl 2-Methyl-2-Propenoate |
| -53 | Acrylic Acid (AA)/Acrylamide/Methacrylamidopropyltrimonium Chloride (MAPTAC) Copolymer |
| -54 | Polymeric quaternary ammonium salt prepared by the reaction of aspartic acid and C6-18 alkylamine with dimethylaminopropylamine and sodium chloroacetate |
| -55 | 1-Dodecanaminium, N,N-Dimethyl-N-[3-[(2-Methyl-1-Oxo-2-Propenyl)AminoPropyl]-, Chloride, Polymer with N-[3-(Dimethylamino)Propyl]-2-Methyl-2-Propenamide and 1-Ethenyl-2-Pyrrolidinone |
| -56 | Polymeric quaternary ammonium salt prepared by the reaction of aspartic acid and C6-18 alkylamine with dimethylaminopropylamine and sodium chloroacetate. |
| -57 | Polymeric quaternary ammonium salt consisting of Castor Isostearate Succinate (q.v.) and Ricinoleamidopropyltrimonium Chloride (q.v.) monomers |
| -58 | 2-Propenoic Acid, Methyl Ester, Polymer with 2,2-Bis[(2-Propenyloxy)Methyl]-1-Butanol and Diethenylbenzene, Reaction Products with N,N-Dimethyl-1,3-Propanediamine, Chloromethane-Quaternized |
| -59 | Polyquaternium polyester |
| -60 | 9-Octadecenoic Acid, 12-Hydroxy-, [(2-Hydroxyethyl)Imino]Di-2,1-Ethanediyl Ester, Polymer with 5-Isocyanato-1-(Isocyanatomethyl)-1,3,3-Trimethylcyclohexane, Compd. with Diethyl Sulfate |
| -62 | Polymeric quaternary ammonium salt prepared by the reaction of butyl methacrylate, polyethylene glycol methyl ether methacrylate, ethylene glycol dimethacrylate and 2-methacryloyethyl trimonium chloride with 2,2'-azobis(2-methyl propionamidine) dihydrochloride |
| -63 | Copolymer of acrylamide, acrylic acid and ethyltrimonium chloride acrylate |
| -65 | Polymeric quaternary ammonium salt consisting of 2-methacryloyloxyethylphosphorylcholine, butyl methacrylate and sodium methacrylate monomers |
| -68 | Quaternized copolymers of vinylpyrrolidone (VP), methacrylamide(MAM) vinylimidazole(VI) & quaternized vinylimidazole (QVI) |

In one or more embodiments, the polyquaternium polymer includes a quaternized copolymer of vinylpyrrolidone and dimethylamino methacrylate, a hydrophobically modified quaternized copolymer of vinylpyrrolidone & dimethylaminopropyl methacrylamide, or a mixture thereof.

In one embodiment, the polyquaternium polymer has a molecular weight of from 1,000 to 5,000,000, in another embodiment, from about 1500 to about 2,500,000 and in yet another embodiment, from about 1,000,000 to about 2,000,000.

Other foam stabilizers that may operate to improve foam quality and/or stability include terpolymers of vinylcaprolactam (VCL), vinylpyrrolidone (VP) and dialkylaminoalkyl acrylate, including a VP/vinylcaprolactam/dimethylaminopropyl methacrylamide copolymer. Yet another foam stabilizer includes isobutylene/dimethylaminopropyl maleimide/ethoxylated maleimide/maleic acid copolymer. These and other foam stabilizers are sometimes referred to as film-forming polymers.

Still other foam stabilizers include acrylamide/ammonium acrylate copolymer, acrylamides/DMAPA acrylates/methoxy PEG methacrylate copolymer, acrylamide/sodium acryloyldimethyltaurate/acrylic acid copolymer, acrylamidopropyltrimonium chloride/acrylamide copolymer, acrylamidopropyltrimonium chloride/acrylates copolymer, acrylates/acetoacetoxyethyl methacrylate copolymer, acrylates/acrylamide copolymer, acrylates/ammonium methacrylate copolymer, acrylates/t-butylacrylamide copolymer, acrylates copolymer, acrylates/C1-2 succinates/hydroxyacrylates copolymer, acrylates/ethylamine oxide methacrylate copolymer, acrylates/lauryl acrylate/stearyl acrylate/ethylamine oxide methacrylate copolymer, acrylates/octylacrylamide copolymer, acrylates/octylacrylamide/diphenyl amodimethicone copolymer, acrylates/polytrimethyl siloxymethacrylate copolymer, acrylates/stearyl acrylate/ethylamine oxide methacrylate copolymer, acrylates/trifluoropropylmethacrylate/polytrimethyl siloxymethacrylate copolymer, acrylates/VA copolymer, acrylates/VP copolymer, adipic acid/diethylenetriamine copolymer, adipic acid/dimethylaminohydroxypropyl diethylenetriamine copolymer, adipic acid/epoxypropyl diethylenetriamine copolymer, adipic acid/isophthalic acid/neopentyl glycol/trimethylolpropane copolymer, allyl stearate/VA copolymer, aminoethylacrylate phosphate/acrylates copolymer, aminoethylpropanediol-acrylates/acrylamide copolymer, aminoethylpropanediol-AMPD-acrylates/diacetoneacrylamide copolymer, ammonium VA/acrylates copolymer, amodimethicone/silsesquioxane copolymer, AMPD-acrylates/diacetoneacrylamide copolymer, AMP-acrylates/allyl methacrylate copolymer, AMP-acrylates/C1-18 alkyl acrylates/C1-8 alkyl acrylamide copolymer, AMP-acrylates/diacetoneacrylamide copolymer, AMP-acrylates/dimethylaminoethylmethacrylate copolymer, bacillus/rice bran extract/soybean extract ferment filtrate, behenyl methacrylate/ethylamine oxide methacrylate copolymer, bis-butyloxyamodimethicone/PEG-60 copolymer, bis-isobutyl PEG-14/amodimethicone copolymer, bis-isobutyl PEG-15/amodimethicone copolymer, butyl acrylate/ethylhexyl methacrylate copolymer, butyl acrylate/hydroxypropyl dimethicone acrylate copolymer, butyl ester of ethylene/MA copolymer, butyl ester of PVM/MA copolymer, calcium/sodium PVM/MA copolymer, chitosan, chitosan lactate, corn starch/acrylamide/sodium acrylate copolymer, dehydroxanthan gum, diethylene glycolamine/epichlorohydrin/piperazine copolymer, dimethicone crosspolymer, dimethicone/silsesquioxane copolymer, diphenyl amodimethicone, ethyl ester of PVM/MA copolymer, ethyltrimonium chloride methacrylate/hydroxyethylacrylamide copolymer, hydrolyzed wheat protein/PVP crosspolymer, hydroxypropyl dimethiconylpropyl acrylates copolymer, hydroxypropyltrimonium hydrolyzed corn starch, isobutylene/ethylmaleimide/hydroxyethylmaleimide copolymer, isobutylene/MA copolymer, isobutylmethacrylate/trifluoroethylmethacrylate/bis-hydroxypropyl dimethicone acrylate copolymer, isopropyl ester of PVM/MA copolymer, lauryl acrylate crosspolymer, lauryl methacrylate/glycol dimethacrylate crosspolymer, lauryl PEG-9 polydimethylsiloxyethyl dimethicone, methacrylic acid/sodium acrylamidomethyl propane sulfonate copolymer, methacryloyl ethyl betaine/acrylates copolymer, methoxy amodimethicone/silsesquioxane copolymer, methoxy PEG-114/polyepsilon caprolactone, myristic/palmitic/stearic/ricinoleic/eicosanedioic glycerides, octylacrylamide/acrylates/butylaminoethyl methacrylate copolymer, PEG-800/polyvinyl alcohol copolymer, PEG/PPG-25/25 dimethicone/acrylates copolymer, PEG-8/SMDI copolymer, polyacrylamide, polyacrylate-6, polyacrylate-8, polyacrylate-9, polyacrylate-15, polyacrylate-16, polyacrylate-17, polyacrylate-18, polyacrylate-19, polybeta-alanine/glutaric acid crosspolymer, polybutylene terephthalate, polyester-1, polyethylacrylate, polyethylene terephthalate, polyimide-1, polymethacryloyl ethyl betaine, polypentaerythrityl terephthalate, polyperfluoroperhydrophenanthrene, polyquaternium-4/hydroxypropyl starch copolymer, polyurethane-1, polyurethane-6, polyurethane-10, polyurethane-18, polyurethane-19, polyvinyl acetate, polyvinyl butyral, polyvinylcaprolactam, polyvinylformamide, polyvinyl imidazolinium acetate, polyvinyl methyl ether, potassium butyl ester of PVM/MA copolymer, potassium ethyl ester of PVM/MA copolymer, PPG-70 polyglyceryl-10 ether, PPG-12/SMDI copolymer, PPG-51/SMDI copolymer, PVM/MA copolymer, PVP/VA/itaconic acid copolymer, PVP/VA/vinyl propionate copolymer, rhizobian gum, rosin acrylate, shellac, silicone quaternium-16/glycidoxy dimethicone crosspolymer, sodium butyl ester of PVM/MA copolymer, sodium ethyl ester of PVM/MA copolymer, sodium polyacrylate, sodium polygamma-glutamate, soy protein phthalate, sterculia urens gum, terephthalic acid/Isophthalic acid/sodium isophthalic acid sulfonate/glycol copolymer, trimethylolpropane triacrylate, trimethylsiloxysilylcarbamoyl pullulan, VA/crotonates copolymer, VA/crotonates/methacryloxybenzophenone-1 copolymer, VA/crotonates/vinyl neodecanoate copolymer, VA/crotonates/vinyl propionate copolymer, VA/DBM copolymer, VA/vinyl butyl benzoate/crotonates copolymer, vinylamine/vinyl alcohol copolymer, vinyl caprolactam/VP/dimethylaminoethyl methacrylate copolymer, VP/acrylates/lauryl methacrylate copolymer, VP/dimethylaminoethylmethacrylate copolymer, VP/DMAPA acrylates copolymer, VP/hexadecene copolymer, VP/methacrylamide/vinyl imidazole copolymer, VP/VA copolymer, VP/vinyl caprolactam/DMAPA acrylates copolymer, yeast palmitate, a silicon-based polymer or resin such as phenylpropyldimethyl siloxysilicate, trimethylsiloxysilicate, cyclopentasiloxane, trimethylsiloxysilicate, diisostearoyl trimethyllolpropane siloxy silicate, vinyl dimethicone crosspoylmer/blends, and alkyl cetearyl dimethicone crosspolymers.

In one embodiment, the foam stabilizer includes a VP/vinylcaprolactam/dimethylaminopropyl methacrylamide copolymer sold under the trade names Aquaflex SF-40, or an isobutylene/dimethylaminopropyl maleimide/ethoxylated maleimide/maleic acid copolymer sold under the trade name Aquaflex XL-30.

In one embodiment, foam stabilizer is present in an amount of from about 0.005 to about 4 weight percent active, based upon the total weight of the alcoholic composition. In another embodiment, the foam stabilizer is present in an amount of from about 0.01 to about 1 weight percent, based upon the total weight of the alcoholic composition, and in yet another embodiment, the foam stabilizer is present in an amount of from about 0.02 to about 0.2 weight percent, based upon the total weight of the alcoholic composition.

In one embodiment, the foam stabilizer is added directly to the alcoholic composition. In one or more other embodiments, the foam stabilizer is added to the alcoholic composition as a solution or emulsion. In other words, the foam stabilizer may be premixed with a carrier to form a foam stabilizer solution or emulsion, with the proviso that the carrier does not deleteriously affect the foaming properties of the alcoholic composition. Examples of carriers include water, alcohol, glycols such as propylene or ethylene glycol, ketones, linear and/or cyclic hydrocarbons, triglycerides, carbonates, silicones, alkenes, esters such as acetates, benzoates, fatty esters, glyceryl esters, ethers, amides, polyethylene glycols and PEG/PPG copolymers, inorganic salt solutions such as saline, and mixtures thereof. It will be understood that, when the foam stabilizer is premixed to form a foam stabilizer solution or emulsion, the amount of solution or emulsion that is added to the alcoholic composition is selected so that the amount of foam stabilizer falls within the ranges set forth hereinabove.

In one embodiment, the weight ratio of foaming surfactant to foam stabilizer is from about 3.5:1 to about 14.5:1, and in another embodiment, the weight ratio of foaming surfactant to foam stabilizer is from about 8:1 to about 11:1.

In certain embodiments, the foam stabilizer increases the foam stability, i.e. the amount of time that it takes for foam to break down into a liquid. Particularly, it has been found that, when the foaming surfactant comprises a fluorosurfactant, the presence of a foam stabilizer improves the stability of the foam. When the foaming surfactant comprises a siloxane polymer, the foam stabilizer is optional.

The foam stabilizer may operate to improve the stability of the foam in any number of ways. In one or more embodiments, the foam stabilizer also improves the foam quality, i.e. increases the number of bubbles and/or reduces the size of the bubbles.

Stabilized foamed compositions, were compared to unstabilized foamed compositions after dispensing the foams onto a surface. Both foamed compositions contained the same amounts of ethanol, fluorosurfactant, humectant, and water. The stabilized foamed composition contained about 0.14 % by weight foam stabilizer. It was found that the unstabilized foam contained some small bubbles, but contained a greater amount of large, loose bubbles than did the stabilized foam.

One minute after the foams were dispensed, it was found that, while the stabilized foam remained essentially unchanged, the unstabilized foam had become watery around the edges.

Three minutes after the foams were dispensed, it was found that the stabilized foam remained essentially unchanged, while the unstabilized foam had become very watery.

Five minutes after the foams were dispensed, it was found that the stabilized foam remained essentially unchanged, while the unstabilized foam was primarily liquid, with only a few bubbles remaining.

One hour after the foams were dispensed, it was found that, while the stabilized foam remained very foamy, the unstabilized foam had collapsed to become a liquid. In embodiments, the stability of the foam produced when a composition comprising, a fluorosurfactant, a foam stabilizer and greater than 40 wt. % alcohol is passed through a non-aerosol foaming pump at room temperature is greater than the stability of the foam produced when a composition not including the foam stabilizer is passed through the non-aerosol foaming pump.

In one embodiment, the foam stability of the foam produced when the foamable alcoholic composition of the present invention is passed through a non-aerosol foaming pump at room temperature is at least about 30 seconds, in another embodiment at least about three minutes, or in other words, the alcoholic composition maintains its foam form and doesn't break down into liquid form for at least three minutes. In another embodiment, the foam stability is at least about five minutes, and in yet another embodiment, the foam stability is at least about 15 minutes. In one or more embodiments, the foam stability is at least about 30 minutes, and in other embodiments, the foam stability is at least about 60 minutes.

The alcoholic composition of this invention may further include a wide range of optional ingredients, with the proviso that they do not deleteriously affect the foam forming properties of the alcoholic composition, or the stability of the foam. The CTFA International Cosmetic Ingredient Dictionary and Handbook, Eleventh Edition, 2005, and the 2004 CTFA International Buyer's Guide, both of which are incorporated by reference herein in their entirety, describe a wide variety of non-limiting cosmetic and pharmaceutical ingredients commonly used in the skin care industry, that are suitable for use in the compositions of the present invention. Non-limiting examples of functional classes of ingredients are described in these references. Examples of these functional classes include: abrasives, anti-acne agents, anticaking agents, antioxidants, binders, biological additives, bulking agents, chelating agents, chemical additives; colorants, cosmetic astringents, cosmetic biocides, denaturants, drug astringents, emulsifiers, external analgesics, film formers, fragrance components, humectants, opacifying agents, plasticizers, preservatives, propellants, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, detackifiers, and viscosity increasing agents (aqueous and nonaqueous). Examples of other functional classes of materials useful herein that are well known to one of ordinary skill in the art include solubilizing agents, sequestrants, and keratolytics, and the like. In one embodiment, the alcoholic composition further comprises glycerin.

Auxiliary surfactants may be included in the alcoholic compositions for the purpose of boosting or modifying the foam quality and characteristics, for modifying the feel of the final formulation during rub in and/or dry time, for providing persistence or long-lasting microbial action of the alcohol, for solubilizing other ingredients such as fragrances or sunscreens, and for irritation mitigation. Auxiliary surfactants include, but are not necessarily limited to, sulfosuccinates, amine oxides, PEG-80 sorbitan laurate, polyglucosides, alkanolamides, sorbitan derivatives, fatty alcohol ethoxylates, quaternary ammonium compounds, amidoamines, sultaines, isothionates, sarcosinates, betaines, and fatty alcohol polyethylene glycols.

Although a propellant may be used to produce stable foam, advantageously a propellant is not necessary. In certain embodiments, the amount of propellant is less than about 1000 parts per million by weight, based upon the total weight of the alcoholic composition. In one embodiment, the alcoholic composition is substantially free of propellants, such as hydrocarbon propellants. By substantially free is meant that the amount of propellant in the alcoholic composition is less than about 100 parts per million by weight, based upon the total weight of the alcoholic composition.

In one embodiment, alcohol is the only active antimicrobial ingredient introduced into the composition, and in this embodiment the amount of auxiliary antimicrobial ingredients is less than about 0.1 weight percent, based upon the total weight of the alcoholic composition. In other embodiments, the composition includes auxiliary antimicrobial agents in addition to alcohol. Examples of auxiliary antimicrobial agents include, but are not limited to, triclosan, also known as 5-chloro-2(2,4-dichlorophenoxy) phenol and available from Ciba-Geigy Corporation under the tradename IRGASAN®; chloroxylenol, also known as 4-chloro-3,5-xylenol, available from Nipa Laboratories, Inc. under the tradenames NIPACIDE® MX or PX; hexetidine, also known as 5-amino-1,3-bis(2-ethylhexyl)-5-methyl-hexahydropyrimidine; chlorhexidine salts including chlorhexidine gluconate and the salts of N,N"-Bis(4-chlorophenyl)-3,12-diimino-2,4,11,14-tetraazatetradecanediimidi amide; 2-bromo-2-nitropropane-1; 3-diol, benzalkonium chloride; cetylpyridinium chloride; alkylbenzyldimethylammonium chlorides; iodine; phenol derivatives, povidone-iodine including polyvinylpyrrolidinone-iodine; parabens; hydantoins and derivatives thereof, including 2,4-imidazolidinedione and derivatives of 2,4-imidazolidinedione as well as dimethylol-5,5-dimethylhydantoin (also known as DMDM hydantoin or glydant); phenoxyethanol; cis isomer of 1-(3-chloroallyl)-3,5,6-triaza-1-azoniaadamantane chloride, also known as quaternium-15 and available from Dow Chemical Company under the tradename DOWCIL™ 2000; diazolidinyl urea; benzethonium chloride; methylbenzethonium chloride; and mixtures thereof. When used, the auxiliary antimicrobial agents are present in amounts of from about 0.1 to about 1 wt. %, based upon the total weight of the alcoholic composition.

The alcoholic composition of the present invention may optionally further comprise a wide range of topical drug actives, with the proviso that they do not deleteriously affect the foam forming properties of the alcoholic composition, or the stability of the foam. Examples of topical drug actives include salicylic acid, acetyl salicylic acid, cis-retinoic acid, trans-retinoic acid, N-acetyl-L-cysteine, lipoic acid, azelaic acid, phytic acid, lisophosphotidic acid, tetracycline, ibuprofen, naproxen, acetominophen, hydrocortisone, resorcinol, phenoxyethanol, phenoxypropanol, phenoxyisopropanol, 2,4,4'-trichloro-2'-hydroxy diphenyl ether, 3,4,4'-trichlorocarbanilide, octopirox, 2-phenylbenzimidazole-5-sulfonic acid, dihydroxyacetone, benzoyl peroxide, 2,4,4'-trichloro-2-hydroxy diphenyl ether, phenoxyethanol, phenoxypropanol, phenoxyisopropanol, phytic acid, lipoic acid, lisophosphatidic acid, benoxaprofen, flubiprofen, fenoprofen, fenbufen, ketoprofen, indoprofen, priprofen, carprofen, oxaprozin, pranoprofen, miroprofen, tioxaprofen, suprofen, alminoprofen, tiaprofenic acid, fluprofen, bucloxic acid, benzocaine, lidocaine, bupivacaine, chloroprocaine, dibucaine, etidocaine, mepivacaine, tetracaine, dyclonine, hexylcaine, procaine, cocaine, ketamine, pramoxine, phenol, dihydroxyacetone, tyrosine, ethyltryosinate, phospho-DOPA, .beta.-lactim drugs, quinoline drugs, ciprofloxacin, norfloxacin, erythromycin, amikacin, doxycycline, capreomycin, chlorhexidine, chlortetracycline, oxytetracycline, clindamycin, ethambutol, hexamidinee isethionate, metronidazole, pentamidine, , gentamicin, kanamycin, lineomycin, methacyclin, methenamine, minocycine, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxyclcyline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amnanfadine sulfate, octopirox, parachlorometa xylenol, nystatin, tolnaftate, clotrimazole, 2-ethylhexyl p-methoxycinnamate, octyl methoxycinnamate, p-amino benzoate, p-aminobenzoic acid, 2-phenyl benzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, silica, iron oxide, 4-N,N-(2-ethylhexyl)methyl aminobenzoic acid ester of 2,4-dihydroxybenzophenone, 4-N,N-(2-ethylhexyl)methyl aminobenzoic acid ester with 4-hydroxydibenzoylmethane, 4-N,N-(2-ethylhexyl)methyl aminobenzoic acid ester of 2-hydroxy-4-(2-hydroxyethoxy)benzophenone, 4-N,N-(2-ethylhexyl)-methyl aminobenzoic acid ester of 4-(2-hydroxyethoxy)dibenzoylmethane, tetracycline, ibuprofen, naproxen, acetaminophen, resorcinol, 3,4,4'-trichlorocarbanilide, octopirox, pharmaceutically-acceptable salts and mixtures of the above.

In one or more embodiments, the balance of the alcoholic composition includes water or other suitable solvent. In one embodiment, one or more volatile silicone-based materials are included in the formulation to further aid the evaporation process. Exemplary volatile silicones have a lower heat of evaporation than alcohol. In certain embodiments, use of silicone-based materials can lower the surface tension of the fluid composition. This provides greater contact with the surface. In one embodiment, the silicone-based material, such as cyclomethicone, trimethylsiloxy silicate or a combination thereof, may be included in the formulation at a concentration of from about 4 wt. % to about 50 wt. % and in another embodiment from about 5 wt. % to about 35 wt. %, and in yet another embodiment from about 11 wt. % to about 25 wt. %, based upon the total weight of the alcoholic composition.

In certain embodiments, such as the rinse-off formulation described above, the balance of the alcoholic composition includes foaming surfactant.

The alcoholic composition may be prepared by simply mixing the components together. The order of addition is not particularly limited. In one embodiment, the skin sanitizing alcoholic composition is prepared by a method comprising dispersing foaming surfactant in alcohol with slow to moderate agitation, adding water, and then adding a foam stabilizer, and mixing until the mixture is homogeneous.

The foamable composition of the present invention may be employed in any type of dispenser typically used for foam products. Advantageously, while the foamable composition can optionally be foamed by aerosolizing the composition, an aerosolized product is not necessary for foaming. Any dispenser that is capable of mixing the foamable alcoholic composition with air or an inert gas may be used. Inert gases include gas that does not substantially react or otherwise deleteriously affect the foamable composition. Examples of inert gases include nitrogen, argon, xenon, krypton, helium, neon, and radon. In one embodiment, the alcoholic composition is used in dispensers that employ foaming pumps, which combine ambient air or an inert gas and the alcoholic composition in a mixing chamber and pass the mixture through a mesh screen. In this and other embodiments, the viscosity of the composition is less than about 100 mPas, in one embodiment less than about 50 mPas, and in another embodiment less than about 25mPas.

The alcoholic composition of the present invention produces stabilized foam. As a result, the foamed composition will be less likely to break down to a liquid state and drip from the foaming pump and/or associated dispenser elements.

Accordingly, the present invention further provides a method of reducing dripping of a composition from a foaming pump. When a foamable composition is dispensed by using a foamable pump, a portion of the dispensed composition, now in foam form, remains in the dispenser head until another aliquot is dispensed. If the foam in the dispenser head breaks down into liquid between uses of the dispenser, the liquid will drip out of the dispenser head. The time between uses of the dispenser can be designated generally as X minutes. "X" can vary widely.

Advantageously, the stabilized foam of the present invention does not drip from the dispenser when X is about 3 minutes or less. In one embodiment, the stabilized foam does not drip from the dispenser when X is about five minutes or less, and in another embodiment, when X is about 15 minutes or less. In one or more embodiments, the stabilized foam does not drip from the dispenser when X is about 30 minutes or less, and in other embodiments, the stabilized foam does not drip from the dispenser when X is about 60 minutes or less.

In order to demonstrate the practice of the present invention, the following examples have been prepared and tested. The examples should not, however, be viewed as limiting the scope of the invention. The claims will serve to define the invention.

### Examples

Example 1 was prepared by dispersing the fluorosurfactant, DEA-C8-18 perfluoroalkylether phosphate, in ethanol with slow to moderate agitation until a homogeneous dispersion was achieved. Next, humectant was added and mixed until the mixture was homogeneous. Water was added, with mixing. The solution was agitated until a homogeneous mixture was achieved.

The foamable mixture was passed through an Airspray® foaming pump. The foam was rated for quality and stability as described hereinbelow, and the results are summarized in Table 2.

A simple 1 -5 rating system was used to rate the foam of each system, with 5 being the best.

Foam Quality Rating Scale: Quality is a measurement of the visible appearance of the dispensed foam when dispensed from a typical commercial foaming pump.
Scale 0 - 5
0 No foam
1 Watery, weak foam with large loose bubbles
2 Large, loose bubbles, some small bubbles
3 Foam has about equal amounts of smaller and larger bubbles
4 Foam has primarily small, tight bubbles
5 Creamy, dense foam with small, tight bubbles

Foam Stability Rating Scale. Stability was rated by measuring the time it takes for the foam to breakdown into a liquid. A poor stability rating correlates to dripping from a wall or table top dispenser. Ratings of 3 or higher show considerable stabilization of the alcohol foam.
Scale 0 - 5
0 Immediately melts (<30 seconds)
1 Melts in about 30-60 seconds
2 Melts in about 1-15 minutes
3 Melts in about 15-30 minutes
4 Melts in about 30-60 minutes
5 Foam remains after about 60 minutes

Example 2 was prepared as described for Example 1, except that polyquaternium 11 was added after the water, and agitated until a homogeneous mixture was achieved. The foamable mixture was passed through a foaming pump and rated as for Example 1. The results are summarized in Table 2. The term "qs" indicates a sufficient amount to total 100 percent.

Examples 3-7 were prepared, foamed, and rated as described for Examples 1 and 2, except that amounts were varied as shown in Table 2.

**Table 2**

| **Ingredient (weight percent)** | **Comments** | **Ex. 1** | **Ex. 2** | **Ex. 3** | **Ex. 4** | **Ex. 5** | **Ex. 6** | **Ex. 7** |
|---|---|---|---|---|---|---|---|---|
| Water | | qs | qs | qs | qs | qs | qs | qs |
| Ethanol | | 65.50 | 65.50 | 65.50 | 65.50 | 65.50 | 65.50 | 65.50 |
| DEA-C8-18 Perfluoroalkylethyl Phosphate | 15wt. % in water | 2.25 | 2.25 | 4.00 | 4.00 | 4.00 | 3.00 | 4.00 |
| Humectant | | 2.50 | 2.50 | 3.00 | 3.00 | 3.00 | 3.00 | 3.00 |
| Polyquaternium-11 | 20wt. % in water | 0 | 0.40 | 0 | 0.10 | 0.20 | 0.20 | 0.30 |
| Total | | 100 | 100 | 100 | 100 | 100 | 100 | 100 |
| | | | | | | | | |
| **Foam Quality Rating** | | 3 | 3 | 4 | 4 | 5 | 5 | 5 |
| **Foam Stability Rating** | | 2 | 5 | 2 | 3 | 5 | 5 | 5 |

Examples 8 - 11 were prepared as described for Example 2, except that in Examples 8, 9, and 11, polyquaternium 11 was replaced by other polymers. Each foamable mixture was passed through a foaming pump and rated as for Example 1. The results are summarized in Table 3.

**Table 3**

| **Ingredient (weight percent)** | **Comment** | **Ex. 8** | **Ex. 9** | **Ex. 10** | **Ex. 11** |
|---|---|---|---|---|---|
| Water | | qs | qs | qs | Qs |
| Ethanol | | 65.50 | 65.50 | 65.50 | 65.50 |
| DEA-C8-18 Perfluoroalkylethyl Phosphate | 15wt. % in water | 2.25 | 2.25 | 2.25 | 4.00 |
| Humectant | | 2.50 | 2.50 | 2.50 | 3.00 |
| Polyvinyl pyrrolidone | 95wt. % in water | 0.80 | -- | -- | -- |
| Isobutylene/dimethylaminopropyl maleimide/ethoxylated maleimide/maleic acid copolymer | 30wt. % in ethanol | -- | 0.10 | -- | -- |
| Quaternized copolymer of vinylpyrrolidone & dimethylaminopropyl methacrylamide (polyquaternium 11) | 20wt. % in water | -- | -- | 0.56 | -- |
| VP/Vinylcaprolactam/DMAPA Acrylates Copolymer | 40wt. % in ethanol | | | | 0.50 |
| **Total** | | 100 | 100 | 100 | 100 |
| | | | | | |
| **Foam Quality Rating** | | 3 | 3 | 4 | 4 |
| **Foam Stability Rating** | | 2 | 5 | 5 | 5 |

Examples 12 - 24 were prepared as described for Example 1, except that the amounts of fluorosurfactant were varied, and two foaming surfactants, perfluorononylethyl carboxydecal peg10 dimethicone, and dimethicone copolymer undecylenate, were used in addition to or in place of the fluorosurfactant of Example 1. Example 10 also contains polyquaternium 11. Each foamable mixture was passed through a foaming pump and rated as for Example 1. The results are summarized in Tables 4 and 5.

**Table 4**

| **Ingredient** | **Comment** | **Ex. 12** | **Ex. 13** | **Ex. 14** | **Ex. 15** | **Ex. 16** | **Ex. 17** |
|---|---|---|---|---|---|---|---|
| Water | | 28.3 | 28.3 | 28.3 | 28.3 | 28.3 | 31.5 |
| Ethanol | | 65.5 | 65.5 | 65.5 | 65.5 | 65.5 | 65.5 |
| DEA C8-C18 perfluoroalkylethyl Phosphate | 15wt. % in water | 1.5 | 0.167 | 0.5 | 1.0 | 0.33 | 0.5 |
| Humectant | | 3 | 3 | 3 | 3 | 3 | -- |
| Perfluorononylethyl Carboxydecal PEG-10 Dimethicone | 100 | 1.5 | 2.83 | 2.5 | 2.0 | 2.67 | 2.5 |
| Polyquaternium 11 | 20wt. % in water | 0.2 | 0.2 | 0.2 | 0.2 | 0.2 | -- |
| | | | | | | | |
| | | | | | | | |
| **TOTAL** | | 100 | 100 | 100 | 100 | 100 | 100 |
| Foam quality rating | | 4 | 3 | 3 | 3 | 3 | 1 |
| Foam stability rating | | 3 | 2 | 3 | 3 | 2 | 2 |

**Table 5**

| **Ingredient** | **Comment** | **Ex. 18** | **Ex. 19** | **Ex. 20** | **Ex. 21** | **Ex. 22** | **Ex. 23** | **Ex. 24** |
|---|---|---|---|---|---|---|---|---|
| Water | | 31.5 | 30.3 | 31.8 | 33.3 | 31.5 | 31 | 31.5 |
| Ethanol | | 65.5 | 65.5 | 65.5 | 65.5 | 65.5 | 65.5 | 65.5 |
| DEA C8-C18 perfluoroalkylethyl Phosphate | 15wt. % in water | -- | 3.0 | 1.0 | 0.5 | -- | 1.0 | 0.75 |
| Humectant | | -- | -- | 1.0 | -- | -- | 1.0 | 1.0 |
| Perfluorononylethyl Carboxydecal PEG-10 Dimethicone | 100 | 3.0 | 1.0 | -- | -- | 3.0 | -- | -- |
| Dimethicone PEG-7 undecylenate | 100 | -- | -- | -- | -- | -- | 1.0 | 0.75 |
| Polyquaternium 11 | 20wt. % in water | -- | 0.2 | 0.7 | 0.7 | -- | 0.5 | 0.5 |
| | | | | | | | | |
| | | | | | | | | |
| **TOTAL** | | 100 | 100 | 100 | 100 | 100 | 100 | 100 |
| Foam quality rating | | 1 | 3 | 3 | 3 | 3 | 3 | 3 |
| Foam stability rating | | 2 | 4 | 3 | 3 | 2 | 4 | 3 |

Examples 25 - 36 were prepared as described for Example 2, except that the amounts were varied as shown in Tables 6 and 7, and in some examples, various foaming surfactants were used in addition to or in place of the fluorosurfactant of Example 2. Example 33 does not contain polyquaternium 11. The results are summarized in Tables 6 and 7.

**Table 6**

| **Ingredient** | **Comment** | **Ex. 25** | **Ex. 26** | **Ex. 27** | **Ex. 28** | **Ex. 29** | **Ex. 30** |
|---|---|---|---|---|---|---|---|
| Water | | 28.2 | 29.3 | 28.2 | 28.2 | 28.2 | 29.2 |
| Ethanol | | 65.5 | 65.5 | 65.5 | 65.5 | 65.5 | 65.5 |
| DEA C8-C18 perfluoroalkylethyl Phosphate | 15wt. % in water | -- | 1 | 0.5 | 1 | 0.25 | -- |
| PEG-10 dimethicone | | 3 | 1 | 2.5 | 2 | 2.75 | 2 |
| Humectant | | 3 | 3 | 3 | 3 | 3 | 3 |
| Fragrance | | 0.075 | 0.075 | 0.075 | 0.075 | 0.075 | 0.075 |
| Polyquaternium 11 | 20wt. % in water | 0.2 | 0.2 | 0.2 | 0.2 | 0.2 | 0.2 |
| **TOTAL** | | 100 | 100 | 100 | 100 | 100 | 100 |
| Foam quality rating | | 4 | 5 | 5 | 5 | 4 | 3 |
| Foam stability rating | | 3 | 5 | 5 | 5 | 5 | 3 |

**Table 7**

| **Ingredient** | **Comment** | **Ex. 31** | **Ex. 32** | **Ex. 33** | **Ex. 34** | **Ex. 35** | **Ex. 36** |
|---|---|---|---|---|---|---|---|
| Water | | 31.2 | 32.2 | 28.4 | 28.7 | 31 | 30.7 |
| Ethanol | | 65.5 | 65.5 | 65.5 | 65.5 | 65.5 | 65.5 |
| DEA C8-C18 perfluoroalkylethyl Phosphate | 15wt. % in water | -- | -- | -- | 0.5 | 0.25 | 0.5 |
| PEG-10 dimethicone | | 2 | 1 | 3 | 2 | 2 | 2 |
| Humectant | | 1 | 1 | 3 | 3 | 1 | 1 |
| Fragrance | | 0.075 | 0.075 | 0.075 | 0.075 | 0.075 | 0.075 |
| Polyquaternium 11 | 20wt. % in water | 0.2 | 0.2 | -- | 0.2 | 0.2 | 0.2 |
| **TOTAL** | | 100 | 100 | 100 | 100 | 100 | 100 |
| Foam quality rating | | 3 | 3 | 3 | 5 | 4 | 5 |
| Foam stability rating | | 3 | 3 | 3 | 5 | 5 | 5 |

The foamable composition of Example 1 was placed in an Airspray® foaming pump in a wall dispenser. An aliquot of the composition was dispensed from the pump. After about 30 seconds to 1 minute, liquid was observed dripping from the dispenser head.

To the foamable composition of Example 1 was added 0.1 wt. % polyqaternium 11. This composition was placed in an Airspray® foaming pump in a wall dispenser. An aliquot of the composition was dispensed from the pump. During about the next 10 minutes, no dripping was observed.

Various modifications and alterations that do not depart from the scope and spirit of this invention will become apparent to those skilled in the art. This invention is not to be duly limited to the illustrative embodiments set forth herein.

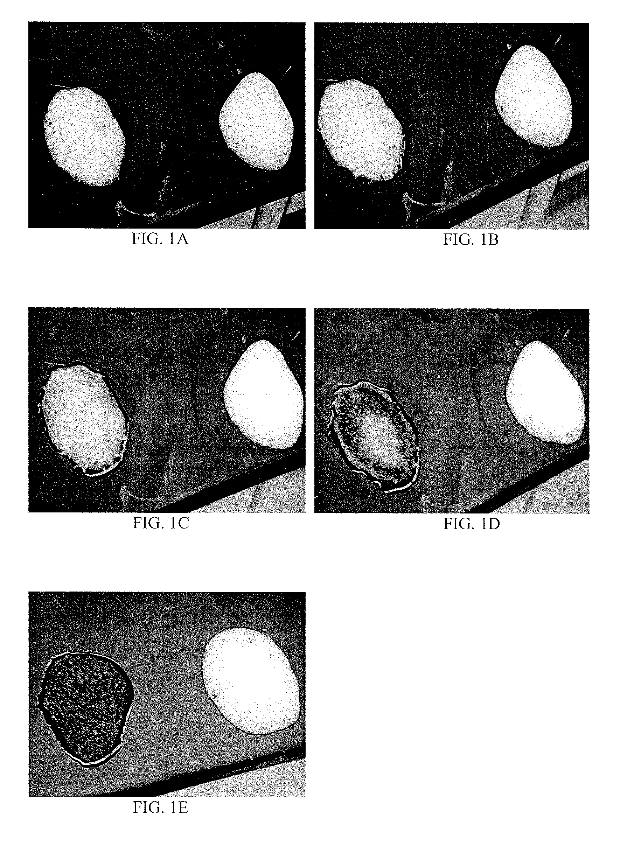

## Claims

1. A foamable alcoholic composition comprising:
at least about 40 weight percent alcohol, based upon the total weight of the alcoholic composition; and
a foaming surfactant selected from the group consisting of siloxane polymer surfactants, anionic, cationic, and zwitterionic fluorosurfactants, and combinations thereof; with the proviso that when the foaming surfactant comprises a fluorosurfactant, the foamable alcoholic composition further comprises a polymeric or oligomeric foam stabilizer, wherein the foamable alcoholic composition has a viscosity of less than about 100 mPas.

2. A method for reducing dripping of a composition from a foaming pump dispenser adapted to dispense said composition, the method comprising:
providing a foamable composition comprising at least about 40 weight percent alcohol, based upon the total weight of the alcoholic composition, and a foaming surfactant selected from the group consisting of siloxane polymer surfactants, anionic, cationic, and zwitterionic fluorosurfactants, and combinations thereof, with the proviso that where the foaming surfactant comprises a fluorosurfactant, the foamable alcoholic composition further comprises a polymeric or oligomeric foam stabilizer;
providing a foaming pump dispenser having a dispenser head;
placing the foamable composition into the foaming pump dispenser; and
dispensing an aliquot of said foamable composition, wherein said foamable composition has a viscosity of less than about 100 mPas, wherein a portion of said aliquot remains in the dispenser head for at least about 3 minutes, and wherein said portion remaining in said dispenser head does not drip from the dispenser.

3. The composition of claim 1, or method of claim 2, wherein said foamable composition has a viscosity of less than about 50 mPas.

4. The composition of claim 1, or method of claim 2, wherein said foaming surfactant comprises a fluorosurfactant compound that may be represented by the formula
[F₃CF₂C-(CF₂CF₂)ₓ-CH₂CH₂-O-P₂O₃]⁻ [R¹]⁺
where [R¹] + includes DEA, TEA, NH₄, or betaine, and where x is an integer from about 4 to about 18.

5. The composition of claim 1, or method of claim 2, wherein said foaming surfactant comprises a siloxane polymer surfactant that may be represented by the formula
R² - Si(CH₃)₂-[O-Si(CH₃)₂]*ₐ*-[O-Si(CH₃)R³]*_{b}*-O-Si(CH₃)₂-R²
where R² and R³ independently include a methyl group or a moiety that may be represented by the formula
-(CH₂)₃-O-(CH₂CH₂O)*_{c}*-[CH₂CH(CH₃)O]*_{d}*-(CH₂CH₂O)*ₑ*H
with the proviso that both R² and R³ are not CH₃, where a is an integer from about 3 to about 21, b is an integer from about 1 to about 7, c is an integer from about 0 to about 40, d is an integer from about 0 to about 40, and e is an integer from about 0 to about 40, with the proviso that a ≥ 3 x b and that c + d + e ≥ 5.

6. The composition of claim 1, or method of claim 2, wherein said siloxane polymer surfactant comprises a surfactant selected from the group consisting of organopolysiloxane dimethicone polyols, silicone carbinol fluids, silicone polyethers, alkylmethyl siloxanes, amodimethicones, trisiloxane ethoxylates, dimethiconols, quaternized silicone surfactants, polysilicones, silicone crosspolymers, silicone waxes, and mixtures thereof.

7. The composition of claim 1, or method of claim 2, wherein said siloxane polymer surfactant comprises dimethicone PEG-7 undecylenate, PEG-10 dimethicone, PEG-8 dimethicone, PEG-12 dimethicone, perfluorononylethyl carboxydecal PEG 10, PEG-20/PPG-23 dimethicone, PEG-11 methyl ether dimethicone, bis-PEG/PPG-20/20 dimethicone, silicone quats, PEG-9 dimethicone, PPG-12 dimethicone, fluoro PEG-8 dimethicone, PEG 23/PPG 6 dimethicone, PEG 20/PPG 23 dimethicone, PEG 17 dimethicone, PEG5/PPG3 methicone, bis-PEG20 dimethicone, a PEG/PPG20/15 dimethicone copolyol and sulfosuccinate blend, or a mixture thereof.

8. The composition of claim 1, or method of claim 2, wherein said foam stabilizer comprises a polyquaternium, a quaternized copolymer of vinylpyrrolidone and dimethylamino methacrylate, a hydrophobically modified quaternized copolymer of vinylpyrrolidone & dimethylaminopropyl methacrylamide, or a mixture thereof.

9. The composition of claim 1, or method of claim 2, wherein said foam stabilizer comprises a terpolymer of vinylcaprolactam (VCL), vinylpyrrolidone (VP) and dialkylaminoalkyl acrylate, or a isobutylene/dimethylaminopropyl maleimide/ethoxylated maleimide/maleic acid copolymer.

10. An antimicrobial composition comprising:
at least about 40 weight percent alcohol, based upon the total weight of the alcoholic composition; and
a foaming surfactant selected from the group consisting of siloxane polymer surfactants, anionic, cationic, and zwitterionic fluorosurfactants, and combinations thereof; with the proviso that when the foaming surfactant comprises a fluorosurfactant, the foamable alcoholic composition further comprises a polymeric or oligomeric foam stabilizer, wherein the antimicrobial composition has a viscosity of less than about 100 mPas.
